# EUROPÄISCHE PATENTSCHRIFT

(11) **EP 0 803 502 B1**
(45) Veröffentlichungstag und Bekanntmachung des Hinweises auf die Patenterteilung: **26.06.2002**
(21) Anmeldenummer: 97106476.1
(22) Anmeldetag: 18.04.1997
(51) Int. Cl.: C07D 241/04, B01J 31/22

(54) **Verfahren zur Herstellung von optisch aktiven 2-Piperazincarbonsäurederivaten**
Process for the preparation of optically active piperazine-2-carboxylic acid derivatives
Procédé de préparation de dérivés d'acide 2-pipérazine-carboxylique optiquement actifs

(30) Priorität: 23.04.1996 CH 102896
(43) Veröffentlichungstag der Anmeldung: 29.10.1997
(73) Patentinhaber: Lonza AG, 3930 Visp (CH)
(72) Erfinder: Fuchs, Rudolf, Dr., 1950 Sion (Kanton Wallis) (CH)
(74) Vertreter: Winter, Brandl, Fürniss, Hübner, Röss, Kaiser, Polte Partnerschaft

(56) Entgegenhaltungen:
- EP-A- 0 680 955
- EP-A- 0 744 401
- WO-A-95/21162
- GB-A- 2 302 690
- CHEMICAL ABSTRACTS, vol. 124, no. 13, 1996 Columbus, Ohio, US; abstract no. 176155x, Seite 1303; Spalte 2; XP002041827 & JP 07 291 943 A (KOEI)
- K.ROSSEN ET AL.: "ASYMETRIC HYDROGENATION OF TETRAHYDROPYRAZINES:" TETRAHEDRON LETTERS., Bd. 36, Nr. 36, 1995, OXFORD GB, Seiten 6419-6422, XP002041826

## Beschreibung

Die vorliegende Erfindung betrifft ein Verfahren zur Herstellung von optisch aktiven 2-Piperazincarbonsäurederivaten, insbesondere von Estern und Amiden der (*R*)- oder (*S*)-2-Piperazincarbonsäure, die durch die allgemeine Formel 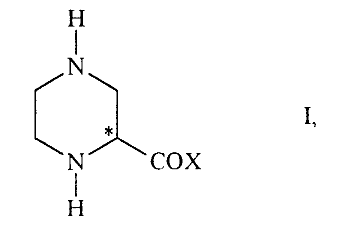 worin X eine Hydroxygruppe, eine C₁₋₆-Alkoxygruppe oder eine Gruppe der Formel -NR¹R² ist, worin wiederum
(i) R¹ und R² unabhängig voneinander Wasserstoff, C₁₋₆-Alkyl, C₃₋₆-Cycloalkyl, Aryl oder eine Aminoschutzgruppe bedeuten oder
(ii) R¹ und R² zusammen mit dem Stickstoffatom einen gegebenenfalls substituierten 5- oder 6-gliedrigen gesättigten heterocyclischen Ring bilden,
repräsentiert werden, durch asymmetrische Hydrierung der entsprechenden Pyrazincarbonsäurederivate. Verbindungen dieser Gruppe sind wertvolle Zwischenprodukte, insbesondere für die Herstellung von pharmazeutischen Wirkstoffen. So sind beispielsweise einige bekannte Verbindungen mit X = NH-*t*-Bu und (*S*)-Konfiguration Bausteine für HIV-Protease-Inhibitoren (EP-A 541 168).

Hier und im folgenden sind unter C₁₋ₙ-Alkyl jeweils geradkettige oder verzweigte primäre, sekundäre oder tertiäre Alkylgruppen mit 1 bis n Kohlenstoffatomen zu verstehen, also beispielsweise unter C₁₋₆-Alkyl, Gruppen wie Methyl, Ethyl, Propyl, Butyl, Pentyl, Hexyl, Isopropyl, Isobutyl, *tert*-Butyl, Isopentyl und Neopentyl. Entsprechend sind unter C₁₋ₙ-Alkoxy die aus C₁₋ₙ-Alkyl und Sauerstoff zusammengesetzten Gruppen zu verstehen. Unter Aryl sind insbesondere Gruppen wie Phenyl oder substituiertes Phenyl zu verstehen. Mögliche Substituenten im "substituierten Phenyl" sind C₁₋₆-Alkyl, fluoriertes C₁₋₆-Alkyl wie beispielsweise Trifluormethyl, Hydroxy, C₁₋₆-Alkoxy, Halogen (insbesondere F und Cl), Amino, (C₁₋₆-Alkyl)amino, Di(C₁₋₆-alkyl)amino, (C₁₋₆-Alkoxy)carbonyl oder allgemein alle Substituenten, die unter den Reaktionsbedingungen nicht verändert oder abgespalten werden und den Katalysator nicht vergiften. Es können auch mehrere (gleiche oder verschiedene) Substituenten vorhanden sein. Unter C₃₋₆-Cycloalkyl sind Cyclopropyl, Cyclobutyl, Cyclopentyl oder Cyclohexyl zu verstehen. Unter Aminoschutzgruppen sind die in der Peptidchemie üblichen Schutzgruppen wie beispielsweise Benzyloxycarbonyl ("Z") oder *tert*-Butoxycarbonyl ("Boc") zu verstehen. Der Ausdruck "gesättigter heterocyclischer Ring" umfasst auch Ringe mit mehreren, gegebenenfalls auch mehreren verschiedenen Heteroatomen wie beispielsweise Morpholin.

Bisher bekannte Synthesen von Verbindungen der Formel I gehen von der Stammverbindung (X = OH) aus, die durch klassische Racematspaltung in optisch aktiver Form aus dem Racemat erhalten werden kann (E. Felder et al., *Helv. Chim. Acta* **1960,** *43,* 888-896). Zunächst werden die Ringstickstoffatome geschützt und dann die freie Carboxylgruppe in das Amid übergeführt. Das Verfahren ist allenfalls für den Labormassstab geeignet und erfordert die Verwendung teurer Reagenzien. Aufgabe der vorliegenden Erfindung war daher, ein auch in technischem Massstab durchführbares Verfahren bereitzustellen, das ohne Racematspaltung auskommt.

Eine asymmetrische Hydrierung von Tetrahydropyrazincarbonsäurederivaten wird in Tetrahedron Letters, 1995, 36, 6419-6422 beschrielsen.

Erfindungsgemäss wird diese Aufgabe durch das Verfahren gemäss Patentanspruch 1 gelöst.

Es wurde gefunden, dass sich die den Zielverbindungen entsprechenden Pyrazincarbonsäurederivate der allgemeinen Formel 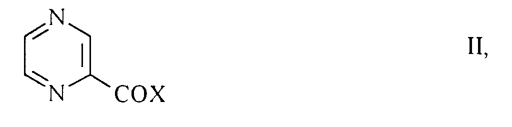 worin X die oben genannte Bedeutung hat, mit Wasserstoff in Gegenwart von katalytisch wirksamen optisch aktiven Rhodiumkomplexen asymmetrisch zu den Zielverbindungen hydrieren lassen.

Vorzugsweise werden als Pyrazincarbonsäurederivate die Amide eingesetzt, in denen X eine Gruppe der Formel -NR¹ R² ist und R¹ und R² die obenstehenden Bedeutungen haben.

Besonders bevorzugt sind solche Amide, in denen R¹ Wasserstoff und R² eine C₁₋₆-Alkylgruppe, insbesondere eine *tert*-Butylgruppe ist.

Die Pyrazincarbonsäurederivate II sind bekannte Verbindungen oder aus den kommerziell erhältlichen Verbindungen Pyrazincarbonsäure, Pyrazincarbonsäureamid und Pyrazincarbonitril nach bekannten Methoden erhältlich.

Als katalytisch wirksamer optisch aktiver Rhodiumkomplex wird vorzugsweise ein Rhodiumkomplex eingesetzt, der durch Reaktion eines Rh(I)-Komplexes mit einem optisch aktiven Metallocenylphosphin gebildet wird.

Als optisch aktive Metallocenylphosphine werden vorzugsweise Verbindungen der allgemeinen Formel 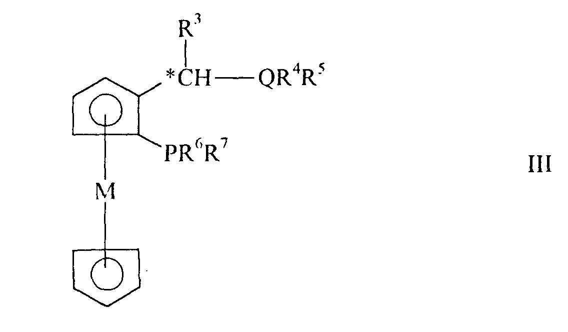 worin M Eisen(II) oder Ruthenium(II), Q Stickstoff oder Phosphor, R³ eine C₁₋₄-Alkylgruppe und R⁴ bis R⁷ unabhängig voneinander C₁₋₈-Alkyl, C₅₋₈-Cycloalkyl, Phenyl oder substituiertes Phenyl bedeuten, eingesetzt.

Besonders bevorzugt sind die Metallocenylphosphine, in denen M Eisen(II) ist, d. h. die Ferrocenylphosphine.

Ebenfalls besonders bevorzugt sind die Metallocenylphosphine, in denen Q Phosphor ist, also Metallocenyldiphosphine.

Weiterhin besonders bevorzugt sind Metallocenylphosphine, in denen R³ Methyl ist und R⁴ und R⁵ gleich sind und *tert*-Butyl oder Cyclohexyl bedeuten. Zu diesen letztgenannten Metallocenylphosphinen zählen beispielsweise das 1-[1-(Di*-tert*-butylphosphino)ethyl]-2-(diphenylphosphino)ferrocen und das 1-[1-(Dicyclohexylphosphino)ethyl]-2-(diphenylphosphino)-ferrocen. Die Herstellung dieser Verbindungen ist in EP-A 0 564 406 beschrieben.

Weitere optisch aktive Metallocenylphosphine sind beispielsweise in T. Hayashi et al., *J. Am. Chem. Soc.* **1994,** *116*, 4221-4226, in EP-A 0 612 758 und in T. Hayashi et al., *Bull. Chem. Soc. Jpn.* **1980,** *53*, 1138-1151 beschrieben.

Als Rh(I)-Komplexe, welche mit den optisch aktiven Metallocenylphosphinen zusammen die katalytisch wirksamen optisch aktiven Rhodiumkomplexe bilden, werden vorzugsweise neutrale zweikernige Komplexe der allgemeinen Formel

[Rh(L)A]₂ IV

eingesetzt. Hierbei steht L für ein C₄₋₁₂-Dien oder zwei C₂₋₁₂-Alkenmoleküle. A bedeutet Chlor, Brom oder Iod, vorzugsweise Chlor oder Brom.

Ebenfalls bevorzugt sind Rh(I)-Komplexe der allgemeinen Formel

[Rh(L)₂]⁺ B⁻ V

worin L die oben genannte Bedeutung hat und B⁻ das Anion einer Sauerstoffsäure oder Komplexsäure ist. Unter Anionen von Sauerstoffsäuren sind beispielsweise Anionen wie ClO₄⁻, SO₃F⁻, CH₃SO₃⁻ oder CF₃SO₃⁻ zu verstehen, unter Anionen von Komplexsäuren solche wie beispielsweise BF₄⁻, PF₆⁻, AsF₆⁻, SbF₆⁻ oder SbCl₆⁻.

Besonders bevorzugt als Liganden L in den Rh(I)-Komplexen IV und V sind Diene, insbesondere Norbornadien und 1,5-Cyclooctadien. Diese Komplexe sind bekannt, beispielsweise aus EP-A 0 302 021 oder US-A 011 995.

Die asymmetrische Hydrierung der Pyrazincarbonsäurederivate II wird vorteilhaft bei einer Temperatur von 20°C bis 200°C und einem Wasserstoffdruck von 1 bis 200 bar durchgeführt. Das molare Verhältnis Katalysator-Edukt liegt vorteilhaft bei 1:5 bis 1:2000, vorzugsweise bei 1:20 bis 1.100
Als Lösungsmittel für die asymmetrische Hydrierung eignen sich beispielsweise Wasser, niedrige Alkohole wie Methanol, aromatische Kohlenwasserstoffe wie Toluol, Ketone wie Aceton oder Carbonsäureester wie Ethylacetat.

Die nachfolgenden Beispiele verdeutlichen die Durchführung des erfindungsgemässen Verfahrens und die Herstellung der erfindungsgemässen Verbindungen, ohne dass darin eine Einschränkung zu sehen ist.

### Beispiel 1

### Pyrazincarbonsäuremethylester

Zu 1200 ml Methanol wurden unter Argon bei 4-6 °C innerhalb von 1 h 106,6 g Thionylchlorid zugetropft. Bei 9 °C wurden 100,1 g Pyrazincarbonsäure zugegeben und das Gemisch 2 h auf 61 °C erwärmt, wobei die Säure vollständig in Lösung ging.
Nach dem Abkühlen auf Raumtemperatur wurde langsam eine Lösung von 145 g Natriumhydrogencarbonat in 1,4 l Wasser zugegeben. Das Methanol wurde am Rotationsverdampfer bei 45 °C Badtemperatur und 50-120 mbar abdestilliert und der Rückstand dreimal mit Dichlormethan (400 ml, 100 ml, 100 ml) extrahiert. Durch Einengen der organischen Phase erhielt man 83,15 g Rohprodukt, das aus ca. 250 g Diisopropylether umkristallisiert wurde. Ausbeute: 70,6 g, zuzüglich 10,28 g aus der Mutterlauge (total 72,5%)
- ¹H NMR (CDCl₃, 400 MHz): δ =: 4,08 (s, 3H);
8,75 (d, *J* = 0,5 Hz, 1H);
8,80 (d, *J* = 0,5 Hz, 1H);
9,30 (s, 1H).

### Beispiel 2

### Pyrazincarbonsäure-tert-butylester

Ein Gemisch von 20 ml *N,N*-Dimethylformamid und 50 ml Acetonitril wurde auf-20 °C gekühlt und bei dieser Temperatur unter Argon innerhalb von 20 min tropfenweise mit einer Lösung von 11,16 g (80 mmol) Oxalylchlorid in 10 ml Acetonitril versetzt. Dann wurden 10 g (80 mmol) Pyrazincarbonsäure (Fluka) bei -20 °C portionsweise zugegeben. Bei dieser Temperatur wurde das Gemisch noch 20 min gerührt und tropfenweise mit 20 ml (212 mmol) *tert*-Butylalkohol in 20 ml Pyridin versetzt. Die Kühlung wurde entfernt und das Reaktionsgemisch bei Raumtemperatur über Nacht stehengelassen. Anschliessend wurde das Gemisch mit 300 ml Dichlormethan und 300 ml einer 20%igen wässrigen Kaliumhydrogencarbonatlösung geschüttelt. Die Phasen wurden getrennt und die wässrige Phase nochmals mit Dichlormethan extrahiert. Aus den vereinigten organischen Phasen wurde durch Abdestillieren des Lösungsmittels die Titelverbindung als Öl erhalten.
Ausbeute: 9,02 g (62%), Gehalt (GC) 99%

### Beispiel 3

### Pyrazincarbonsäure-tert-butylamid

Zu 900 ml Essigsäure wurden bei 20 °C (Kühlung) zuerst innerhalb von 35 min 575 g (5,75 mol) Schwefelsäure und dann innerhalb von 20 min 200,1 g (1,9 mol) Cyanpyrazin getropft. Dann wurden innerhalb von 3,5 h 142,4 g (2,54 mol) Isobuten eingeleitet, wobei die Temperatur durch entsprechende Kühlung unter 25 °C gehalten wurde. Anschliessend wurde das Gemisch noch 2 h bei Raumtemperatur gerührt und dann auf 1,8 l Eiswasser gegossen. Durch langsame Zugabe von 1527 g 30%iger Natronlauge wurde das Gemisch auf pH 3,5 gestellt und dann 4 h im Flüssig-Flüssig-Extraktor mit 2,5 l heissem Methylcyclohexan kontinuierlich extrahiert. Der Extrakt wurde unter Rühren auf 4 °C abgekühlt, das auskristallisierte Produkt abfiltriert und im Vakuum getrocknet.
Ausbeute: 246 g, Gehalt (Titration) 99,6%
Aus der eingeengten Mutterlauge wurden weitere 22,5 g Produkt mit einem Gehalt von 99,0% isoliert.
Gesamtausbeute: 268,5 g (78%)

### Beispiel 4

### (S)-Piperazin-2-carbonsäure-tert-butylamid

In einem Autoklaven wurden unter Argon 0,5 g (2,7 mmol) Pyrazincarbonsäure-*tert*-butylamid (hergestellt nach Beispiel 3), 26,9 mg (58 µmol) Bicyclo[2.2.1]hepta-2,5-dienrhodium(I)chlorid-Dimer und 72 mg (121 µmol) 1-[1(*R*)-(Dicyclohexylphosphino)ethyl]-2(*S*)-(diphenylphosphino)ferrocen vorgelegt. Nach Zugabe von 10 ml entgastem Methanol wurde der Autoklav dreimal mit Argon und zweimal mit Wasserstoff gespült. Dann wurden 50 bar Wasserstoff aufgepresst. Bei 70 °C wurde 20 h hydriert. Der Autoklav wurde entspannt und mit Stickstoff gespült. Das Lösungsmittel wurde vollständig abdestilliert.
Ausbeute: 0,50 g
Mittels NMR wurde der Umsatz auf 80% bestimmt. Der Enantiomerenüberschuss (ee) betrug nach GC-Analyse 77,6%.

### Beispiel 5

### (S)-Piperazin-2-carbonsäure-tert-butylamid

Es wurde verfahren wie in Beispiel 4 beschrieben, jedoch wurden nur 25,3 mg (54 µmol) Bicyclo[2.2.1]hepta-2,5-dienrhodium(I)chlorid-Dimer und anstelle von 1-[1(*R*)-(Dicyclohexylphosphino)ethyl]-2(*S*)-(diphenylphosphino)ferrocen 61,5 mg (113,3 µmol) 1-[1(*R*)-(Di-*tert*butylphosphino)ethyl]-2(*S*)-(diphenylphosphino)ferrocen eingesetzt.
Mittels NMR wurde der Umsatz auf 97% bestimmt. Der Enantiomerenüberschuss (ee) betrug nach GC-Analyse 57,6%.

### Beispiel 6

### (S)-Piperazin-2-carbonsäure-tert-butylamid

Analog zu Beispiel 4 wurden 5 g (27 mmol) Pyrazincarbonsäure-*tert*-butylamid, 127 mg (0,27 mmol) Bicyclo[2.2.1]hepta-2,5-dienrhodium(I)chlorid-Dimer und 353 mg (0,594 mmol) 1-[1(*R*)-(Dicyclohexylphosphino)ethyl]-2(*S*)-(diphenylphosphino)ferrocen in 70 ml Methanol bei 100 °C und 50 bar 20 h hydriert.
Mittels NMR wurde der Umsatz auf 85% bestimmt. Der Enantiomerenüberschuss (ee) betrug nach GC-Analyse 49%.

### Beispiel 7

### (S)-Piperazin-2-carbonsäure-tert-butylester

Analog zu Beispiel 4 wurden 0,52 g (2,85 mmol) Pyrazincarbonsäure-*tert*-butylester (hergestellt nach Beispiel 2), 25,3 mg (54 µmol) Bicyclo[2.2.1]hepta-2,5-dienrhodium(I)chlorid-Dimer und 61,5 mg (113,3 µmol) 1-[1(*R*)-(Di-*tert*-butylphosphino)ethyl]-2(*S*)-(diphenylphosphino)ferrocen in 10 ml Methanol bei 70 °C und 50 bar 20 h hydriert.
Mittels NMR wurde der Umsatz auf 67% bestimmt. Zu Bestimmung des ee-Wertes wurde der Ester hydrolysiert (siehe Beispiel 8).

### Beispiel 8

### (S)-Piperazin-2-carbonsäure-Dihydrochlorid

Der rohe (*S*)-Piperazin-2-carbonsäure-*tert*-butylester aus Beispiel 7 wurde in 2,9 g Wasser und 2,1 g (18,4 mmol) 32%iger Salzsäure 20 min bei 100 °C gerührt. Das Gemisch wurde auf 0 °C abgekühlt und noch 1 h gerührt. Das ausgefallene Hydrochlorid wurde abfiltriert und mit 10 ml Dichlormethan gewaschen.
Ausbeute: 0,24 g (41%, bezogen auf Pyrazincarbonsäure-*tert*-butylester)
ee-Wert: 77,6%

### Beispiel 9

### (S)-Piperazin-2-carbonsäure-methylester

Analog zu Beispiel 4 wurden 0,52 g (3,5 mmol) Pyrazincarbonsäuremethylester (hergestellt nach Beispiel 1), 33,6 mg (72,8 µmol) Bicyclo[2.2.1]hepta-2,5-dienrhodium(I)chlorid-Dimer und 92,9 mg (171 µmol) 1-[1(*R*)-(Di-*tert*-butylphosphino)ethyl]-2(*S*)-(diphenylphosphino)-ferrocen in 10 ml Methanol bei 70 °C und 50 bar 20 h hydriert.
Mittels NMR wurde der Umsatz auf 85% bestimmt. Zu Bestimmung des ee-Wertes wurde der Ester nach Beispiel 8 hydrolysiert.
ee-Wert: 3,6%

## Patentansprüche

1. Verfahren zur Herstellung von optisch aktiven 2-Piperazincarbonsäurederivaten der allgemeinen Formel 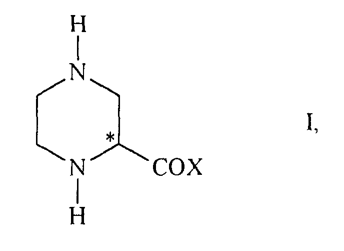 worin X eine Hydroxygruppe, eine C₁₋₆-Alkoxygruppe oder eine Gruppe der Formel -NR¹R² ist, worin wiederum
(i) R¹ und R² unabhängig voneinander Wasserstoff, C₁₋₆-Alkyl, C₃₋₆-Cycloalkyl, Aryl oder eine Aminoschutzgruppe bedeuten oder
(ii) R¹ und R² zusammen mit dem Stickstoffatom einen gegebenenfalls substituierten 5-oder 6-gliedrigen gesättigten heterocyclischen Ring bilden,
**dadurch gekennzeichnet, dass** ein entsprechendes Pyrazincarbonsäurederivat der allgemeinen Formel 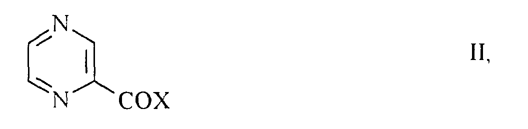 worin X die oben genannte Bedeutung hat, in Gegenwart eines katalytisch wirksamen optisch aktiven Rhodiumkomplexes asymmetrisch hydriert wird.

2. Verfahren nach Anspruch 1, **dadurch gekennzeichnet, dass** als Pyrazincarbonsäurederivat II eine Verbindung eingesetzt wird, in der X eine Gruppe der Formel -NR¹R² ist, worin R¹ und R² die oben genannten Bedeutungen haben.

3. Verfahren nach Anspruch 2, **dadurch gekennzeichnet, dass** R¹ Wasserstoff und R² eine C₁₋₆-Alkylgruppe ist.

4. Verfahren nach Anspruch 3, **dadurch gekennzeichnet, dass** R² eine *tert*-Butylgruppe ist.

5. Verfahren nach einem oder mehreren der Ansprüche 1 bis 4, **dadurch gekennzeichnet, dass** als optisch aktiver Rhodiumkomplex ein aus einem Rh(I)-Komplex und einem optisch aktiven Metallocenylphosphin gebildeter Komplex eingesetzt wird.

6. Verfahren nach Anspruch 5, **dadurch gekennzeichnet, dass** als optisch aktives Metallocenylphosphin eine Verbindung der allgemeinen Formel 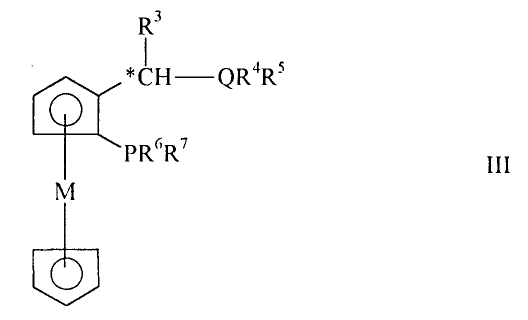 worin M Eisen(II) oder Ruthenium(II), Q Stickstoff oder Phosphor, R³ eine C₁₋₄-Alkylgruppe und R⁴ bis R⁷ unabhängig voneinander C₁₋₈-Alkyl, C₅₋₈-Cycloalkyl, Phenyl oder substituiertes Phenyl bedeuten, eingesetzt wird.

7. Verfahren nach Anspruch 6, **dadurch gekennzeichnet, dass** M Eisen(II) ist.

8. Verfahren nach Anspruch 6 oder 7, **dadurch gekennzeichnet, dass** Q Phosphor ist.

9. Verfahren nach einem der Ansprüche 6 bis 8, **dadurch gekennzeichnet, dass** R³ Methyl ist und R⁴ und R⁵ gleich sind und *tert*-Butyl oder Cyclohexyl bedeuten.

10. Verfahren nach einem oder mehreren der Ansprüche 5 bis 9, **dadurch gekennzeichnet, dass** als Rh(I)-Komplex ein neutraler Komplex der allgemeinen Formel
[Rh(L)A]₂ IV
worin L ein C₄₋₁₂-Dien oder zwei C₂₋₁₂-Alkenmoleküle bedeutet und A Chlor, Brom oder Iod ist, eingesetzt wird.

11. Verfahren nach einem oder mehreren der Ansprüche 5 bis 9, **dadurch gekennzeichnet, dass** als Rh(I)-Komplex ein kationischer Komplex der allgemeinen Formel
[Rh(L)₂]⁺ B⁻ V
worin L ein C₄₋₁₂-Dien oder zwei C₂₋₁₂-Alkenmoleküle bedeutet und B⁻ das Anion einer Sauerstoffsäure oder einer Komplexsäure ist, eingesetzt wird.

12. Verfahren nach Anspruch 10 oder 11, **dadurch gekennzeichnet, dass** L Norbornadien oder 1,5-Cyclooctadien bedeutet.

## Claims

1. A process for the production of optically active 2-piperazinecarboxylic acid derivatives of the general formula 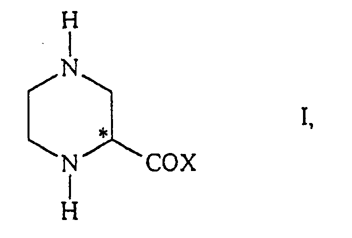 in which X is a hydroxy group, a C₁₋₆ alkoxy group of a group of the formula -NR¹R², in which in turn
(i) R¹ and R² mutually independently mean hydrogen, C₁₋₆ alkyl, C₃₋₆ cycloalkyl, aryl or an amino protective group or
(ii) R¹ and R², together with the nitrogen atom, form an optionally substituted 5- or 6-membered, saturated heterocyclic ring,
**characterised in that** a corresponding pyrazinecarboxylic acid derivative of the general formula 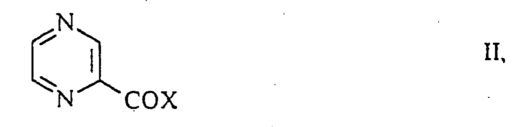 in which X has the above-stated meaning, is asymmetrically hydrogenated in the presence of a catalytically active, optically active rhodium complex.

2. A process according to claim 1, **characterised in that** the pyrazinecarboxylic acid derivative II used is a compound in which X is a group of the formula -NR¹R², in which R¹ and R² have the above-stated meanings.

3. A process according to claim 2, **characterised in that** R¹ is hydrogen and R² a C₁₋₆ alkyl group.

4. A process according to claim 3, **characterised in that** R² is a *tert*.-butyl group.

5. A process according to one or more of claims 1 to 4, **characterised in that** the optically active rhodium complex used is a complex formed from a Rh(I) complex and an optically active metallocenyl phosphine.

6. A process according to claim 5, **characterised in that** the optically active metallocenyl phosphine used is a compound of the general formula 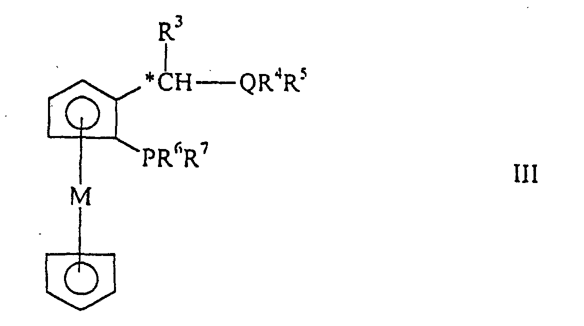 in which M means iron(II) or ruthenium(II), Q means nitrogen or phosphorus, R³ means a C₁₋₄ alkyl group and R⁴ to R⁷ mutually independently mean C₁₋₈ alkyl, C₅₋₈ cycloalkyl, phenyl or substituted phenyl.

7. A process according to claim 6, **characterised in that** M is iron(II).

8. A process according to claim 6 or 7, **characterised in that** Q is phosphorus.

9. A process according to one of claims 6 to 8, **characterised in that** R³ is methyl and R⁴ and R⁵ are identical and mean *tert*.-butyl or cyclohexyl.

10. A process according to one or more of claims 5 to 9, **characterised in that** the Rh(I) complex used is a neutral complex of the general formula
[Rh(L)A]₂ IV
in which L means a C₄₋₁₂ diene or two C₂₋₁₂ alkene molecules and A is chlorine, bromine or iodine.

11. A process according to one or more of claims 5 to 9, **characterised in that** the Rh(I) complex used is a cationic complex of the general formula
[Rh(L)₂]⁺ **B**⁻ V
in which L means a C₄₋₁₂ diene or two C₂₋₁₂ alkene molecules and B⁻ is the anion of an oxo acid or of a complex acid.

12. A process according to claim 10 or 11, **characterised in that** L means norbornadiene or 1,5-cyclooctadiene.

## Revendications

1. Procédé de préparation de dérivés d'acide 2-pipérazinecarboxylique optiquement actifs de formule générale 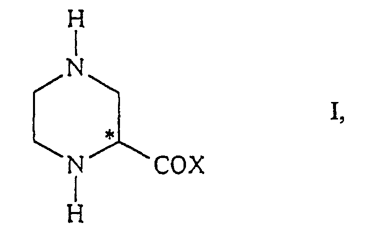 dans laquelle X représente un groupe hydroxy, un groupe alcoxy en C₁-C₆ ou un groupe de formule -NR¹R², dans laquelle
(i) R¹ et R² représentent indépendamment l'un de l'autre un atome d'hydrogène, un groupe alkyle en C₁-C₆, cycloalkyle en C₃-C₆ ou aryle ou un groupe protecteur d'amino,
(ii) R¹ et R² forment ensemble, avec l'atome d'azote, un noyau hétérocyclique saturé de 5 ou 6 chaînons éventuellement substitué,
**caractérisé en ce que** l'on soumet un dérivé d'acide pyrazinecarboxylique correspondant de formule générale 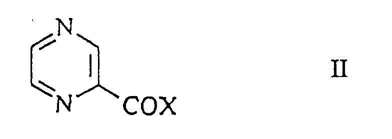 dans laquelle X a la signification indiquée ci-dessus, à une hydrogénation asymétrique en présence d'un complexe de rhodium optiquement actif à activité catalytique.

2. Procédé selon la revendication 1, **caractérisé en ce que** l'on utilise comme dérivé d'acide pyrazinecarboxylique II un composé dans lequel X représente un groupe de formule -NR¹R² dans laquelle R¹ et R² ont les significations indiquées ci-dessus.

3. Procédé selon la revendication 2, **caractérisé en ce que** R¹ représente un atome d'hydrogène et R² un groupe alkyle en C₁-C₆.

4. Procédé selon la revendication 3, **caractérisé en ce que** R² est un groupe tert-butyle.

5. Procédé selon l'une ou plusieurs des revendications 1 à 4, **caractérisé en ce que** l'on utilise comme complexe de rhodium optiquement actif un complexe formé à partir d'un complexe de Rh(I) et d'une métallocénylphosphine optiquement active.

6. Procédé selon la revendication 5, **caractérisé en ce que** l'on utilise comme métallocénylphosphine optiquement active un composé de formule générale 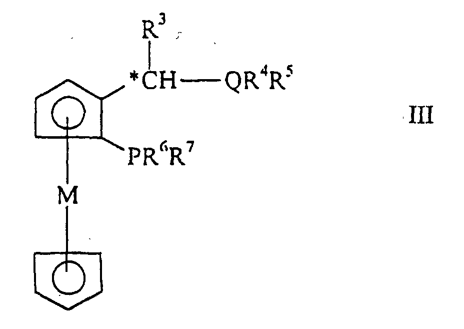 dans laquelle M représente le fer (II) ou le ruthénium (II), Q est un atome d'azote ou de phosphore, R³ représente un groupe alkyle en C₁-C₄ et les R⁴ à R⁷ représentent indépendamment les uns des autres un groupe alkyle en C₁-C₈, cycloalkyle en C₅-C₈, phényle ou phényle substitué.

7. Procédé selon la revendication 6, **caractérisé en ce que** M représente le fer (II).

8. Procédé selon la revendication 6 ou 7, **caractérisé en ce que** Q est un atome de phosphore.

9. Procédé selon l'une des revendications 6 à 8, **caractérisé en ce que** R³ représente un groupe méthyle et R⁴ et R⁵ sont identiques et représentent un groupe tert-butyle ou cyclohexyle.

10. Procédé selon l'une ou plusieurs des revendications 5 à 9, **caractérisé en ce que** l'on utilise comme complexe de Rh(I) un complexe neutre de formule générale
[Rh(L)A]₂ IV
dans laquelle L représente un diène en C₄-C₁₂ ou deux molécules d'alcène en C₂-C₁₂ et A représente un atome de chlore, de brome ou d'iode.

11. Procédé selon l'une ou plusieurs des revendications 5 à 9, **caractérisé en ce que** l'on utilise comme complexe de Rh(I) un complexe cationique de formule générale
[Rh(L)₂]⁺B⁻ V
dans laquelle L représente un diène en C₄-C₁₂ ou deux molécules d'alcène en C₂-C₁₂ et B⁻ représente l'anion d'un acide oxygéné ou d'un acide complexe.

12. Procédé selon la revendication 10 ou 11, **caractérisé en ce que** L représente le norbornadiène ou le 1,5-cyclooctadiène.
